# EUROPEAN PATENT SPECIFICATION

(11) **EP 3 419 591 B1**
(45) Date of publication and mention of the grant of the patent: **16.08.2023**
(21) Application number: 17706497.9
(22) Date of filing: 22.02.2017
(51) Int. Cl.: A61K 8/895, A61K 8/37, A61Q 17/04, A61Q 19/00, C08G 77/20, C08L 83/04

(54) **PROCESS FOR PREPARING A SILICONE ELASTOMER AND PERSONAL CARE COMPOSITION CONTAINING THE ELASTOMER**
VERFAHREN ZUR HERSTELLUNG EINES SILIKONELASTOMERS UND KÖRPERPFLEGEZUSAMMENSETZUNG ENTHALTEND DAS ELASTOMER
PROCÉDÉ DE PRÉPARATION D'UN ÉLASTOMÈRE DE SILICONE ET COMPOSITION DE SOINS PERSONNELS CONTENANT L'ÉLASTOMÈRE

(30) Priority: 25.02.2016 WO PCT/CN2016/074539
(43) Date of publication of application: 02.01.2019
(73) Proprietor: Unilever IP Holdings B.V., 3013 AL Rotterdam (NL); Unilever Global IP Limited, Wirral, Merseyside CH62 4ZD (GB)
(72) Inventor: LIMER, Adam, John, Bebington Wirral Merseyside CH63 3JW (GB); LOU, Anjing, Trumbull Connecticut 06611 (US); DOBKOWSKI, Brian, John, Trumbull Connecticut 06611 (US); ZHAO, Wei, Shanghai 200335 (CN); SONG, Wenhui, Shanghai 200335 (CN)
(74) Representative: James, Helen Sarah
(86) International application number: PCT/EP2017/054061
(87) International publication number: WO 2017/144530

(56) References cited:
- WO-A1-2013/162738
- US-A1- 2003 165 452
- US-A1- 2005 027 051
- US-A1- 2007 142 575
- US-A1- 2007 148 244
- US-A1- 2015 225 507
- US-B1- 6 168 782
- Technical datasheet Andisil RTM XL
- Technical datasheet Andisil RTM VS

## Description

### Field of the invention

The present invention is directed to the polymerization of silicone elastomer and the in-situ entrapment of hydrophobic skin actives within the resulting elastomer. The invention is also directed to the elastomer produced as well as personal care compositions comprising such elastomers whereby the compositions display excellent stability and active performance when compared to formulations traditionally made with active added via batch techniques.

### Background of the invention

Skin, for example, is subject to deterioration through dermatological disorders, environmental abuse (wind, air conditioning, central heating) or through the normal aging process (chronoaging) which may be accelerated by exposure of skin to sun (photoaging). In recent years the demand for personal care compositions and methods for improving the appearance and condition of skin has grown enormously.

It is well known, for example, that presently used sunscreens tend to interact with ingredients in skin care formulations, and this includes interacting with other sunscreens as often seen between UVA and UVB filters. Unfortunately, this leads to a situation where the UV filter activity of the sunscreen agents is reduced during storage or after being applied to the skin. Many efforts have been made to improve active efficacy, like sunscreen photo-stability, by replacing conventional actives with other less effective agents or the addition of active enhancers. However, these methods often result in formulation cost increase and/or active efficiency and consumer perceived sensory benefits.

It is of increasing interest to develop ways to stabilize personal care compositions while simultaneously yielding formulae that result in excellent sensory and active benefits after topical application to skin.

This invention, therefore, is directed to the in-situ entrainment or entrapment of active within a silicone elastomer and during polymerization of the same. The active entrapped elastomer of the present invention is found to improve active stability (like SPF stability) largely when the active entrapped elastomer is incorporated into end use personal care compositions, especially when comparing such end use compositions with similar compositions to which active has been added in a traditional procedure as a bulk ingredient. The unexpected benefits of the present invention are end use personal care compositions, like skin care compositions, that provide superior active efficacy and excellent sensory benefits resulting from active being contained in elastomer both during and after formulating such personal care compositions.

### Additional information

Efforts have been made to enhance active stability by using different approaches. For example, conventional sunscreen actives have been replaced with less effective agents or combined with SPF enhancers as described in U.S. Patent Nos. 8,465,729 and 6,126,925, respectively.

Still other effects have been made to improve active stability by encapsulating the actives in a core shell or structured system. For example, U.S. Patent No. 6,774,179 discloses a method for entrapping actives in core-shell or gel particles to increase active stability in formulations.

Even further, in U.S. Published Patent Application 2008/0199526, disclosed is a method to encapsulate a primary sunscreen in a microcapsule to enhance the sunscreen stability.

In U.S. Patent No. 6,207,717, a process to add oil soluble vitamins to an elastomer is described. While efforts have been made to enhance active efficiency, none of such efforts are free of results that include poor sensory and/or formulation instability. Moreover, none of such efforts describe a method and composition as claimed in this invention.

US 6168782 describes a process which requires converting a hydride into a polymeric surfactant via a reaction with a vinyl terminated PEG. The process requires crosslinking to yield a three-dimensional network of elastomeric silicone polyether, active and oil. Use of a vinyl functionalized silicone for backbone formation is not disclosed or suggested.

### Summary of the invention

In a first aspect, the present invention is directed to a method for making a silicone elastomer according to claim 1.

In a second aspect, the present invention is directed to the silicone elastomer according to claim 6.

In a third aspect, the present invention is directed to an end use composition according to claim 7.

In a fourth aspect, the present invention is directed to a non-therapeutic use according to claim 8.

All other aspects of the present invention will more readily become apparent upon considering the detailed description and examples which follow.

Skin, as used herein, is meant to include skin on the face, neck, chest, back, arms (including underarms), hands, legs, buttocks and scalp. Hair includes hair on the head, and nails include both nails on the feet and hands. Active, as used herein, is meant to include a component that improves a body characteristic after topical application like a skin, hair and/or nail characteristic and/or benefits the same wherein the same can be, and preferably, is an active in a leave-on composition, and most preferably, a cream, lotion, balm, deodorant, or gel as well as a shampoo, conditioner or personal wash composition, including a liquid or solid wash composition. Solvent means a hydrophobic material which is a fluid at room temperature and suitable to dissolve the active targeted for entrapment. Silicone elastomer with entrapped active means silicone elastomer that is cross-linked and has entrapped active and solvent. In the case of solvent, entrapping solvent is synonymous with solvent swelling the elastomer. Such a silicone elastomer comprises molecularly dispersed active in that the elastomer has homogeneously dispersed active and is substantially free of active droplets. Substantially free is defined to mean less than 0.5%, and preferably, less than 0.05%, and most preferably, less than 0.01% by weight of all active in the elastomer with entrapped active appears as droplet. In an especially preferred embodiment, the elastomer comprises no active that appears as droplet.

Hydride functionalized elastomer precursor and vinyl functionalized elastomer precursor may also be referred to as hydride precursor and vinyl precursor, respectively. Comprising as used herein, is meant to include consisting essentially of and consisting of. The silicone elastomer of this invention may, therefore, consist essentially of the polymerization product of hydride functionalized silicone elastomer precursor and vinyl functionalized silicone elastomer precursor, active and solvent. For the avoidance of doubt, the precursors of the silicone elastomers made in this invention do not comprise oxygen to oxygen bonds and the resulting silicone elastomers with entrapped active and solvent are non-emulsifying elastomers. Emulsion, as used herein, includes water-in-oil, oil-in-water or double emulsions. Oil-in-water emulsions are typically preferred. Catalyst, as used herein, refers to a solution with 0.25 to 5%, and preferably, 0.25 to 4%, and most preferably, 0.3 to 3.5% active (e.g., metal) in the solution, based on total weight of the catalyst solution and including all ranges subsumed therein.

Except in the operating and comparative examples, or where otherwise explicitly indicated, all numbers in this description indicating amounts or ratios of materials or conditions or reaction, physical properties of materials and/or use are to be understood as modified by the word "about". All percentages in the specification and examples are intended to be by weight unless stated otherwise.

### Detailed description of the preferred embodiments

The hydride functionalized elastomer precursor consists of at least one backbone unit of the formulae: 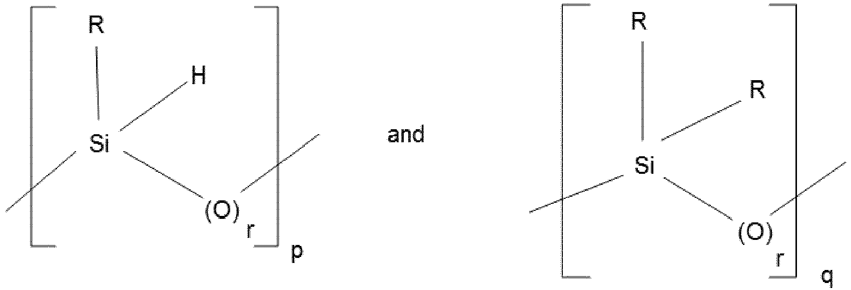 and terminal groups of the formulae: 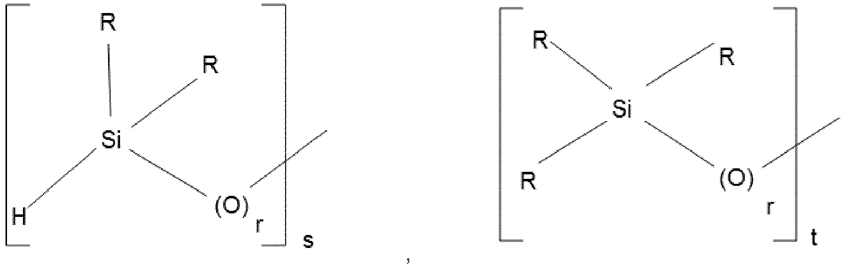 wherein: each R is independently a C₁₋₆ alkyl or aryl (preferably a methyl group);
each r is individually 0 when backbone terminates with oxygen and 1 when backbone terminates with silicon;
p is 0 to 50, q is 2 to 250, s is 0 to 2, t is 0 to 2, s+t=2, p and s are not simultaneously 0, p+q ≥ 1 and p+s is at least 2 (preferably 2 to 15, and most preferably, 3 to 10).

In an often preferred embodiment, p is 2 to 40, and preferably, 10-30, including all ranges subsumed therein. In another often preferred embodiment, q is 2 to 200, and preferably, 15 to 160, including all ranges subsumed therein.

The vinyl functionalized elastomer precursor consists of as blocks or randomly dispersed therein at least one backbone unit of the formulae: 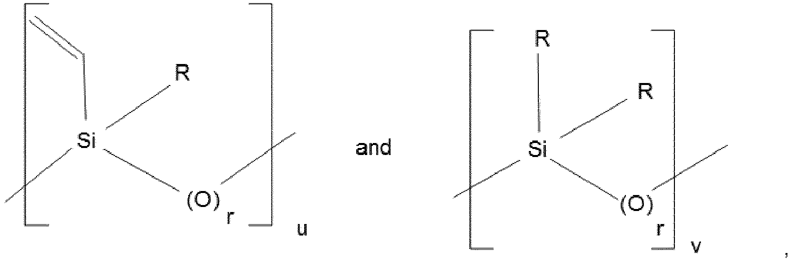 and terminal groups of the formulae: 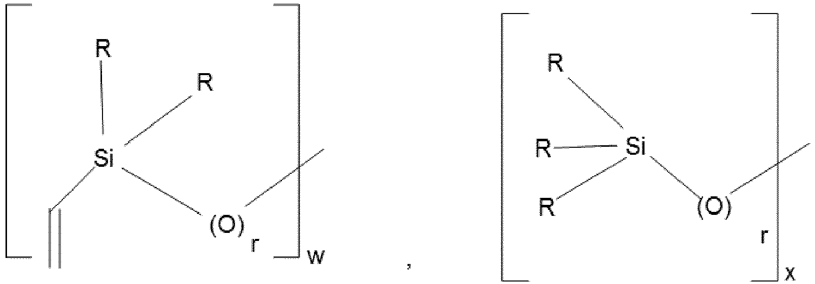 wherein: each R and r are as previously defined, u is 0 to 50, v is 5 to 2,500, w is 0 to 2, x is 0 to 2, w+x=2, u and w are not simultaneously 0, u+v ≥ 1 and u+w is at least 2 (preferably 2 to 15, and most preferably, 2 to 10).

In an often preferred embodiment, u is 0 to 40, and preferably, 0 to 30, including all ranges subsumed therein. In yet another often preferred embodiment, v is 5 to 2250, and preferably, 30 to 1750, including all ranges subsumed therein.

Illustrative examples of the hydride functionalized silicone elastomer precursors that may be used in this invention include Andisil^{®} XL-12, XL-13 and XL-15 (AB Specialty Chemicals) as well as HMS-301 made available from Gelest, Inc. or the like. Illustrative examples of the vinyl functionalized silicone elastomer precursors that may be used in this invention include Andisil VS-6, VS-10, VS-20, VS-50, VS-100, VS-200, VS-250 (AB Specialty Chemicals) as well as DMS-V21 made available from Gelest, Inc. or the like.

Typically, when making the silicone elastomer with entrapped active as described in this invention, the weight ratio of hydride functionalized silicone elastomer precursor (hf) to vinyl functionalized silicone elastomer precursor (vf) is greater than 0.015, and preferably, greater than 0.025, and most preferably from 0.035 to about 0.75, including all ranges subsumed therein. In an often desired embodiment, the ratio of hf/vf is from 0.045 to 0.5, including all ranges subsumed therein.

In another desired embodiment, from 0.05 to 6%, and preferably, from 0.1 to 5%, and most preferably, from 0.1 to 4% by weight hydride functionalized silicone elastomer precursor is used in the method (and product) of this invention based on total weight of hydride precursor, vinyl precursor, active, solvent and catalyst used to make the silicone elastomer with entrapped active, including all ranges subsumed therein.

In yet another desired embodiment, from 5 to 50%, and preferably, from 6 to 40%, and most preferably, from 10 to 25% by weight vinyl functionalized silicone elastomer precursor is used in the method (and product) of this invention based on total weight of hydride precursor, vinyl precursor, active, solvent and catalyst used to make the silicone elastomer with entrapped active, including all ranges subsumed therein.

The solvent suitable for use in this invention may also be used as the cosmetically acceptable carriers suitable for use in end use compositions that comprise the silicone elastomers with entrapped active of this invention. Such solvent/carriers may include mineral oils, silicone oils, synthetic or natural esters, and alcohols. In the end use compositions amounts of these materials may range from 0.1 to 50%, and preferably, from 0.1 to 30%, and most preferably, from 1 to 20% by weight of the composition, including all ranges subsumed therein. In the silicone elastomer made according to this invention (i.e., the ingredient to be used in an end use consumer product), solvent typically makes up from 1 to 96%, and preferably, 2 to 80%, and most preferably, from 3 to 75% by weight of the total weight of the silicone elastomer with entrapped active and solvent, including all ranges subsumed therein. In an especially desired embodiment from 30 to 70% by weight solvent is used based on total weight of the silicone elastomer with entrapped active and solvent, including all ranges subsumed therein. The aforementioned amounts also represent the amounts used in the method for making the silicone elastomer with entrapped active of this invention. For the avoidance of doubt, in the addition/vinyl polymerization carried out to make the silicone elastomer with entrapped active of this invention, solvent is also entrapped with the active in the resulting elastomer to swell the elastomer.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from about 3 to about 9, and preferably, from about 4 to about 5 silicon atoms.

Linear volatile silicone materials generally have viscosities of less than about 5 centistokes at 25°C while cyclic materials typically have viscosities of less than about 10 centistokes.

Nonvolatile silicone oils useful as carrier material that are distinct from the reactants used to synthesize inventive elastomer polymer include polyalkyl siloxanes, polyalkylaryl siloxanes, aryl modified silicones (especially phenyl modified di- and trimethicones) and polyether siloxane copolymers. The essentially non-volatile polyalkyl siloxanes useful herein include, for example, polydimethylsiloxanes (like dimethicone) with viscosities of from about 5 to about 100,000 centistokes at 25°C. Silicone oils (especially, Dimethicones like C6 to C22 alkyl dimethicone) suitable for use are often made commercially available from Dow Corning are preferred.

Among suitable esters are:
(1) Alkenyl or alkyl esters of fatty acids having 6 to 30 carbon atoms like isopropyl palmitate, isopropyl isostearate, isononyl isonanonoate, oleyl myristate, isopropyl myristate, oleyl stearate, and oleyl oleate;
(2) Ether-esters such as fatty acid esters of ethoxylated fatty alcohols;
(3) Polyhydric alcohol esters such as ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1 ,3-butylene glycol monostearate, 1 ,3-butylene glycol distearate, polyoxyethylene polyol fatty acid ester, sorbitan fatty acid esters, and polyoxyethylene sorbitan fatty acid esters;
(4) Ethers including C₆ to C₃₀ ethers like dicaprylyl ether; and
(5) Sterol esters, of which soya sterol and cholesterol fatty acid esters are examples thereof.

Often preferred solvents are polydimethylsiloxane (like Xiameter X-200, 5cst, made commercially available from Dow Corning), cyclodimethylsiloxane, di and/or trimethicones, dicaprylyl ether or blends or mixtures thereof. To the extent such solvents are modified, they are typically phenyl group modified and/or modified with C_{6 to} C₃₀, and preferably, with C₆ to C₂₂ alkyl groups. Particularly preferred for use as a solvent is caprylyl trimethicone like Silsoft 034 made commercially available from Momentive.

Illustrative examples of the actives suitable for use to embed in the elastomers include vitamins like Vitamin A, D, E (and its oil soluble derivatives) and K, sunscreens like methoxycinnamate, ethylhexylmethoxycinnamate, octyl methoxycinnamate, bis-ethyl hexyloxyphenol methoxyphenyl triazine, drometrizole trisiloxane and mixtures thereof.

Other actives that are oil soluble for embedding in the elastomer include resorcinols like 4-ethyl resorcinol, 4-hexyl resorcinol, 4-phenylethyl resorcinol, 4-cyclopentyl resorcinol, 4-cyclohexyl resorcinol, hydroxyacids, mixtures thereof and the like.

Typically, what is used in the process and the amount of active within the silicone elastomer with entrapped active suitable for use in an end use consumer product is from 0.25 to 50%, and preferably, from 2 to 35%, and most preferably, from 10 to 25% by weight based on total weight of silicone elastomer comprising entrapped active and including all ranges subsumed therein.

The catalyst suitable for use in this invention preferably is a transition metal catalyst like vanadium Oxide, iron, manganese oxide and especially platinum catalysts like Platinum (0)-1,3-divinyl-1,1,3,3-tetramethyldisiloxane. Such a catalyst is limited only to the extent that it enhances polymerization of the hydride and vinyl precursors described herein. An effective amount of catalyst is used to enhance polymerization. Typically, from 0.00001 to 0.04% catalyst is used, and preferably, 0.00001 to 0.02%, and most preferably, from 0.0001 to 0.004% by weight catalyst is used, based on total weight of the precursors, solvent, active and catalyst and including all ranges subsumed therein.

In the end use composition comprising the silicone elastomer with entrapped active, typically such composition comprises from 1 to 55%, and preferably, from 1 to 45%, and most preferably, from 2 to 35% by weight silicone elastomer with entrapped active, based on total weight of the end use composition and including all ranges subsumed therein. Such end use compositions may be topically applied (e.g., to hair, nails and skin) and washed off or left on, depending on the end use composition selected for use.

The end use personal care composition comprising the silicone elastomer with embedded active of this invention typically comprises from 0 to 95% water, and preferably, from 3 to 85% and most preferably from 10 to 75% by weight water based on total weight of the end use composition and including all ranges subsumed therein.

Emulsifiers are preferably present in the end use composition containing the inventive elastomer of the present invention. Total concentration of the emulsifier may range from 0.1 to 12%, and preferably, from 1 to 9%, and most preferably, from 1 to 6% by weight of the composition, including all ranges subsumed therein. The emulsifier may be selected from the group consisting of anionic, nonionic, cationic and amphoteric actives. Particularly preferred nonionic actives are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di- fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic emulsifiers.

Preferred anionic emulsifiers include alkyl ether sulfate and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, alkylethercarboxylates and combinations thereof.

Cationic emulsifiers that may be used include, for example, palmitamidopropyltrimonium chloride, distearyldimonium chloride and mixtures thereof. Useful amphoteric emulsifiers include cocoamidopropyl betaine, C₁₂-C₂₀ trialkyl betaines, sodium lauroamphoacetate, and sodium laurodiamphoacetate or a mixture thereof.

Other generally preferred emulsifiers include glyceryl stearate, glycol stearate, stearamide AMP, PEG-100 stearate, cetyl alcohol as well as emulsifying/thickening additives like hydroxyethylacrylate/sodium acryloyldimethyl taurates copolymer/squalane and mixtures thereof.

Emulsion stabilizers generally classified as vegetable based liquids may also be used in the end use compositions. Preferred stabilizers are sold under the name Oilwax LC and made available commercially by Lotioncrafter.

Preservatives can desirably be incorporated into the end use compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are iodopropynyl butyl carbamate, phenoxyethanol, methyl paraben, propyl paraben, imidazolidinyl urea, sodium dehydroacetate and benzyl alcohol. The preservatives should be selected having regard for the use of the personal care composition and possible incompatibilities between the preservatives and other ingredients in the emulsion. Additional preservatives are preferably employed in amounts ranging from 0.01 % to 2% by weight of the composition, including all ranges subsumed therein.

Thickening agents may optionally be included in such end use personal care compositions. Particularly useful are the polysaccharides. Examples include starches, natural/synthetic gums and cellulosics. Representative of the starches are chemically modified starches such as sodium hydroxypropyl starch phosphate and aluminum starch octenylsuccinate. Tapioca starch is often preferred. Suitable gums include xanthan, sclerotium, pectin, karaya, arabic, agar, guar, carrageenan, alginate and combinations thereof. Suitable cellulosics include hydroxypropyl cellulose, hydroxypropyl methylcellulose, ethylcellulose and sodium carboxy methylcellulose. Synthetic polymers are yet another class of effective thickening agent. This category includes crosslinked polyacrylates such as the Carbomers, polyacrylamides such as Sepigel^{®} 305 and taurate copolymers such as Simulgel EG^{®} and Aristoflex^{®} AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100.

Amounts of the thickener, when used, may range from 0.001 to 5%, and preferably, from 0.1 to 3%, and most preferably, from 0.2 to 1.5% by weight of the end use composition including all ranges subsumed therein.

Conventional humectants may be employed in the end use compositions. These are generally polyhydric alcohol-type materials. Typical polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. Most preferred is glycerin, propylene glycol or a mixture thereof. The amount of humectant employed may range anywhere from 0.5 to 20%, preferably between 1 and 15% by weight of the end use composition.

Fragrances, colorants, fixatives and abrasives may optionally be included in end use compositions of the present invention. Each of these substances may range from about 0.05 to about 5%, preferably between 0.1 and 3% by weight.

Azelaic acid, ubiquinone, dihydroxyacetone water (DHA) and mixtures thereof may also be used as actives in the end use composition of this invention apart from the actives in the inventive elastomer. Such compounds, when used, typically make up from 0.2 to 4.5%, and preferably, from 0.5 to 3% by weight of the end use composition, including all ranges subsumed therein.

Desquamation promoters may be present in the end use compositions together with the inventive elastomer. Illustrative are the alpha-hydroxycarboxylic acids, beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic and its derivatives, lactic and malic acids. Salicylic acid is representative of the beta-hydroxycarboxylic acids. Amounts of these materials when present may range from 0.01 to 15% by weight of the end use composition.

A variety of herbal extracts may optionally be included in the personal compositions together with the inventive elastomer. The extracts may either be water soluble or water-insoluble carried in a solvent which respectively is hydrophilic or hydrophobic. Water and ethanol are the preferred extract solvents. Illustrative extracts include those from green tea, yarrow, chamomile, licorice, aloe vera, grape seed, citrus unshui, willow bark, sage, thyme and rosemary. Soy extracts may be used and especially when it is desirable to include retinol.

Conventional buffers/pH modifiers may be used apart from the inventive elastomer and in the end use compositions of this invention. These include commonly employed additives like sodium hydroxide, potassium hydroxide, hydrochloric acid, citric acid and citrate/citric acid buffers. In an especially preferred embodiment, the pH of the end use composition of this invention is from 4 to 8, and preferably, from 4.25 to 7.75, and most preferably, from 5.5 to 7.5, including all ranges subsumed therein. The end use composition of this invention may be a solid stick or bar. Viscosity of the end use composition of this invention is, however, preferably from 1,000 to 120,000 cps, and most preferably, from 5,000 to 80,000 cps taken at ambient temperature and a shear rate of 1s⁻¹ with a strain controlled parallel plate rheometer made commercially available from suppliers like T.A. Instruments under the Ares name.

When making the silicone elastomers with entrapped hydrophobic actives, in no particular order and before polymerization begins, hydride precursor, vinyl precursor, active, solvent and catalyst are combined. At least 20% by weight of the total amount of solvent, and preferably, from 45 to 85%, and most preferably, from 55 to 75% by weight of the total amount of solvent (including all ranges subsumed therein) should be added initially and until polymerization is almost completed (i.e., 80 to 99%, preferably 90 to 99% of the hydride precursor being polymerized). Subsequent to polymerization being almost complete, the remainder of solvent (up to 80% of the total solvent used in the method) should be added (after polymerization is almost completed) gradually for further swelling of the elastomer and typically within 5 to 30 minutes, and preferably, within 8 to 26 minutes, and most preferably, within 10 to 20 minutes so that the resulting silicone elastomer with entrapped active and solvent has a viscosity from 50 to 3,000 cps, and preferably from about 100 to 2,000 cps, and most preferably, from 500 to about 1,600 cps, including all ranges subsumed therein where viscosity is determined with a strain controlled parallel plate rheometer as previously described.

The temperature at which the polymerization reaction takes place ranges from 15 to 75°C, and preferably, from 20 to 70°C, and most preferably from 30 to 65°C, including all ranges subsumed therein. Mixing should occur with moderate shear and under atmospheric conditions.

Surprisingly, the silicone elastomer with entrapped active of the present invention is stable for at least three days, preferably 5 days, and most preferably, for at least 7 days after being stored at 40°C where stable is defined to mean remaining homogeneous, at least translucent (slightly turbid, and deplete of visual separation and active droplet formation).

The silicone elastomer of the present invention has a G' storage module from 700 to 10,000 Pa, and preferably, from 750 to 8,000 Pa, and most preferably, from 775 to 2,500 Pa, including all ranges subsumed therein. In an often desired embodiment, G' storage modules for the silicone elastomers of the present invention is from 800 to 1,500, including all ranges subsumed therein (G' storage modulus obtained by Dynamic Mechanical Analysis, Standard ASTM 4065, from strain sweep at 1 Hz, parallel plates with 25 mm diameter and 0.5 mm gap on an Anton Paar Phisica MCR301 apparatus operating at room temperature).

A wide variety of packaging can be employed to store and deliver the end use composition of this invention. Preferably the package should be able to contain or prevent any elevated pressure build-up during storage and use of the product. Pump dispensers configured to either prevent or withstand high pressure build-up, may be used.

Packaging is often dependent upon the type of personal care composition. For instance, leave-on skin lotions and creams, shampoos, conditioners and shower gels generally employ plastic containers with an opening at a dispensing end covered by a closure. Typical closures are screw-caps, non-aerosol pumps and flip-top hinged lids. Packaging for antiperspirants, deodorants and depilatories may involve a container with a roll-on ball on a dispensing end. Alternatively these types of personal care products may be delivered in a stick composition formulation in a container with propel-repel mechanism where the stick moves on a platform towards a dispensing orifice. Metallic cans pressurized by a propellant and having a spray nozzle serve as packaging for antiperspirants, shave creams and other personal care products. Toilette bars may have packaging constituted by a cellulosic or plastic wrapper or within a cardboard box or even encompassed by a shrink wrap plastic film.

The following examples are provided to facilitate an understanding of the present invention. The examples are not intended to limit the scope of the claims.

### Example 1

Elastomer compositions consistent with this invention have been prepared. Active was added before polymerization was initiated.

| Material | Sample 1 | Sample 2 | Sample 3 |
|---|---|---|---|
| Silicone Hydride(1) | 1.70q | 1.70g | 1.70q |
| Vinyl Silicone(2) | 16.94g | 16.94g | 16.94g |
| PDMS 5cst 25°C(3) | 33.18g | 30.68g | 28.18g |
| Caprylyl trimethicone(4) | 33.18g | 30.68g | 28.18g |
| Ethylhexyl Methoxycinnamate (5) | 15.00g | 20.00g | 25.00g |
| Solvent, 1:1 PDMS 5cst and caprylyl trimethicone* | 34.6g | 34.6g | 34.6g |
| Appearance at room temperature | Translucent | Translucent | Slightly turbid |
| G' @ 0.1% Strain Pa | 1047 | 1105 | 1085 |

| | | | |
|---|---|---|---|
| 1)AB Silicones, Waukegan Illinois, Andisil XL-15 (hydride functionalized silicone elastomer precursor) 2)AB Silicones, Waukegan Illinois, Andisil VS-200 (vinyl functionalized silicone elastomer precursor) 3)Dow Corning, Xiameter X-200 5cst (polydimethylsiloxane) 4)Momentive, Silsoft 034 5)DSM, Parsol MCX *solvent added post polymerization, at least 34% of the total solvent used in the process | | | |

The elastomer was prepared as follows:
Caprylyl trimethicone and PDMS (no more than 66% of the total amount of solvent used) and ethylhexyl methoxycinnamate were combined in a 250ml dry flask and mixed until homogeneous. Vinyl silicone and silicone hydride were added and the resulting mixture was heated to 45° C with reflux of water. Stirring was maintained at 200 rpm with an anchor stirrer. The platinum complex catalyst (divinyltetramethylsiloxane complex, 3 to 3.5% by weight platinum, made commercially available from Gelest, Inc.), 50ul, was added and the reaction stirred for 5 hours while maintained at 45°C. The resulting gelled mixture was diluted with the remainder of solvent which was added dropwise via syringe over a 15 minute period. The obtained sample was characterized to obtain storage modulus G'.

The results obtained surprisingly show silicone elastomers with entrapped active having an excellent G' storage modulus where the elastomers were free of visual separation after being storage at 40°C for 7 days.

### Example 2

Comparative examples were prepared by the following procedure:

| Material | Comparative 1 | Comparative 2 | Comparative 3 |
|---|---|---|---|
| Silicone Hydride(1) | 1.70g | 1.70g | 1.70g |
| Vinyl Silicone(2) | 16.94g | 16.94g | 16.94g |
| PDMS 5cst 25°C(3) | 41.25g | 41.25g | 41.25g |
| Caprylyl trimethicone(4) | 41.25g | 41.25g | 41.25g |
| Solvent: 1:1 PDMS 5cst and caprylyl trimethicone* | 19.6g | 14.6g | 9.6g |
| Ethylhexyl | | | |
| Methoxycinnamate (5) | 15g | 20g | 25g |
| Appearance at room temperature | Very Turbid | Very Turbid & Phase Separation | Very Turbid & Phase Separation |
| G' @ 0.1% Strain | 567 | 438 | 325 |

| | | | |
|---|---|---|---|
| 1)AB Silicones, Waukegan Illinois, Andisil XL-15 2)AB Silicones, Waukegan Illinois, Andisil VS-200 3)Dow Corning, Xiameter X-200 5cst 4)Momentive, Silsoft 034 5)DSM, Parsol MCX *no more than 19.5% solvent added after polymerization | | | |

Caprylyl trimethicone and PDMS (at least 80.5% solvent), vinyl silicone and silicone hydride were combined in a 250ml dry flask and mixed until homogeneous. The mixture was heated to 45°C with reflux of water and stirring at 200rpm with an anchor stirrer. The catalyst platinum complex (as used in Example 1), 50ul, was added and the reaction and stirred for 5 hours at 45°C. The resulting gelled mixture was diluted with the remainder of solvent (a mixture of Caprylyl trimethicone, PDMS and ethylhexyl methoxycinnamate) was added dropwise via syringe over a 15 minute period. The obtained sample was characterized by rheology test to obtain storage modulus G'.

The results and observations indicate that when active is added after polymerization, a very cloudy heterogenous product with active droplets in the resulting elastomer is formed. After polymerization, a G' of 567 Pa was observed, which is inferior for use in consumer products like topical skin compositions.

The results surprisingly indicate that post addition of active, after polymerization is completed, results in an elastomer that is cloudy and of lower elasticity.

The Samples made in Comparative Example 2 showed separation within 24 hours after being stored at 40°C and the Samples became gritty, making them ineffective for use in personal care compositions.

## Claims

1. A method for making a silicone elastomer with entrapped hydrophobic active, comprising the steps of:
1. combining, in no particular order:
(i) 0.05 to 8% by weight of a hydride functionalized silicone elastomer precursor;
(ii) 2 to 60% by weight of a vinyl functionalized silicone elastomer precursor;
(iii) 0.5 to 97% by weight of a solvent comprising polydimethylsiloxane or caprylyl trimethicone;
(iv) 0.25 to 65% by weight hydrophobic active; and
(v) catalyst at an amount effective to catalyze polymerization of the hydride and vinyl functionalized elastomer precursors; and
2. recovering silicone elastomer with entrapped hydrophobic active, wherein:
(i) the hydrophobic active is soluble in the solvent; and
(ii) at least 20% of total solvent used to make the silicone elastomer is provided before polymerization is initiated wherein any remainder of solvent used is provided gradually within 5 to 30 minutes in further swelling of elastomer when polymerization of the hydride is 90 to 99% complete and the silicone elastomer with entrapped hydrophobic active has a viscosity from 50 to 3000 cps and a G' storage modulus from 700 to 10,000PA
(iii) the hydrophobic active is a sunscreen, oil soluble vitamin, hydroxyacid, or resorcinol; wherein the hydride functionalized elastomer precursor consists of at least one backbone unit of the formulae: 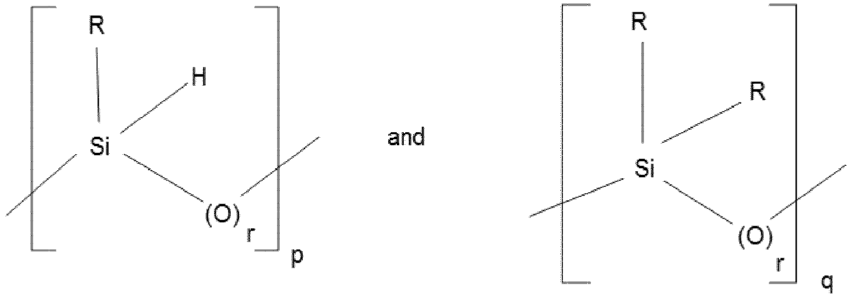 and terminal groups of the formulae: 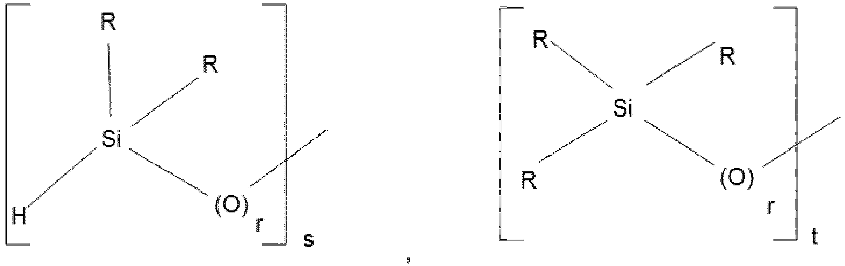 wherein:
a) each R is independently a C₁₋₆ alkyl or aryl;
b) each r is independently 0 when backbone unit terminates with oxygen and 1 when backbone terminates with silicon;
c) p is 0 to 50, q is 2 to 250, s is 0 to 2, t is 0 to 2, s+t=2, p and s are not simultaneously 0, p+q ≥ 1and p+s is at least 2;
and wherein the vinyl functionalized elastomer precursor consists of as blocks or randomly dispersed therein at least one backbone unit of the formulae: 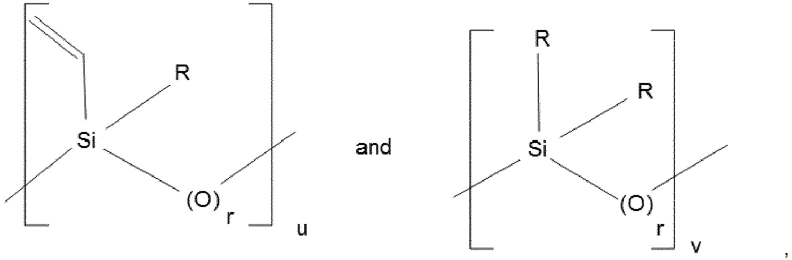 and terminal groups of the formulae: 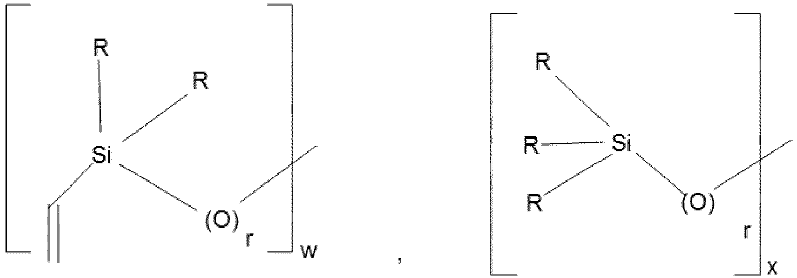 wherein:
a) each R is independently a C₁₋₆ alkyl or aryl;
b) each r is independently 0 when backbone terminates with oxygen and 1 when backbone terminates with silicon;
c) u is 0 to 50, v is 5 to 2,500, w is 0 to 2, x is 0 to 2, w+x=2, u and w are not simultaneously 0, u + v ≥ 1 and u+w is at least 2.

2. The method for making a silicone elastomer according to claim 1 wherein 0.05 to 6% by weight hydride functionalized silicone elastomer precursor is used.

3. The method for making a silicone elastomer according to claims 1 or 2 wherein 5 to 50% by weight vinyl functionalized silicone elastomer precursor is used.

4. The method for making a silicone elastomer according to any one of claims 1 to 3 wherein the weight ratio of hydride functionalized silicone elastomer precursor to vinyl functionalized silicone elastomer precursor is greater than 0.015.

5. The method for making a silicone elastomer according to any one of claims 1 to 4 wherein the method is carried out at a temperature from 15 to 75°C.

6. A silicone elastomer obtainable by the process of any one of claims 1 to 5.

7. An end use composition which is an emulsion comprising from 0.001 to 45% by weight of the silicone elastomer obtainable by the process of any one of claims 1 to 5.

8. Non-therapeutic use of the composition in claim 7 to treat hair, nails and/or skin.

## Patentansprüche

1. Verfahren zur Herstellung eines Silikonelastomers mit eingeschlossenem hydrophobem Wirkstoff, umfassend die Schritte:
1. Kombinieren, in keiner bestimmten Reihenfolge:
(i) 0,05 bis 8 Gewichts-% eines Hydrid-funktionalisierten Silikonelastomer-Vorläufers;
(ii) 2 bis 60 Gewichts-% eines Vinyl-funktionalisierten Silikonelastomer-Vorläufers;
(iii) 0,5 bis 97 Gewichts-% eines Lösungsmittels, umfassend Polydimethylsiloxan oder Caprylyltrimethicon;
(iv) 0,25 bis 65 Gewichts-% hydrophoben Wirkstoff; und
(v) Katalysator in einer Menge, die wirksam ist, um die Polymerisation der Hydrid- und Vinyl-funktionalisierten Elastomer-Vorläufer zu katalysieren; und
2. Rückgewinnen des Silikonelastomers mit eingeschlossenem hydrophobem Wirkstoff, wobei:
(i) der hydrophobe Wirkstoff in dem Lösungsmittel löslich ist; und
(ii) mindestens 20% des gesamten zur Herstellung des Silikonelastomers verwendeten Lösungsmittels vor dem Initiieren der Polymerisation bereitgestellt werden, wobei jeglicher Rest des verwendeten Lösungsmittels graduell innerhalb von 5 bis 30 Minuten bei weiterem Quellen des Elastomers bereitgestellt werden, wenn die Polymerisation des Hydrids zu 90 bis 99% abgeschlossen ist und das Silikonelastomer mit eingeschlossenem hydrophobem Wirkstoff eine Viskosität von 50 bis 3000 cP und einen G'-Speichermodul von 700 bis 10.000 Pa aufweist,
(iii) der hydrophobe Wirkstoff ein Sonnenschutz, ein lösliches Vitamin, Hydroxysäure oder Resorcinol ist; wobei der Hydrid-funktionalisierte Elastomer-Vorläufer aus mindestens einer Grundgerüsteinheit der Formeln besteht: 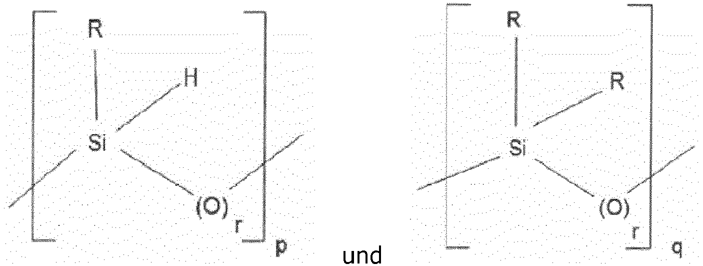 und den Terminalgruppen der Formeln: 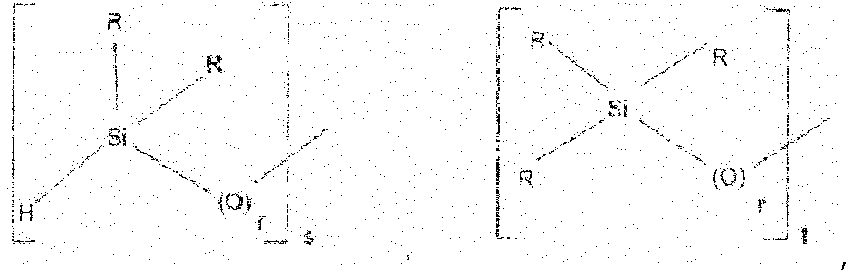 wobei:
a) jedes R unabhängig ein C₁₋₆-Alkyl oder Aryl ist;
b) jedes r unabhängig 0 ist, wenn die Grundgerüsteinheit mit Sauerstoff endet, und 1 ist, wenn das Grundgerüst mit Silikon endet;
c) p 0 bis 50 ist, q 2 bis 250 ist, s 0 bis 2 ist, t 0 bis 2 ist, s+t=2, p und s nicht gleichzeitig 0 sind, p+q ≥ 1 und p+s mindestens 2 ist;
und wobei der Vinyl-funktionalisierte Elastomer-Vorläufer besteht in Form von Blöcken oder mindestens einer darin wahllos verteilten Grundgerüsteinheit der Formeln 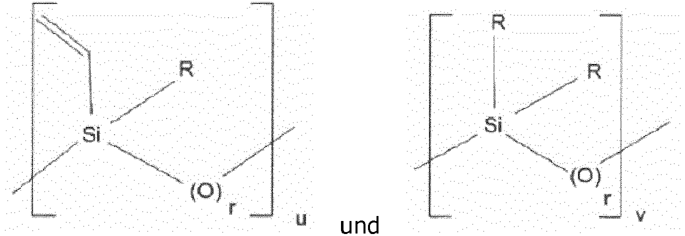 und den Terminalgruppen der Formeln: 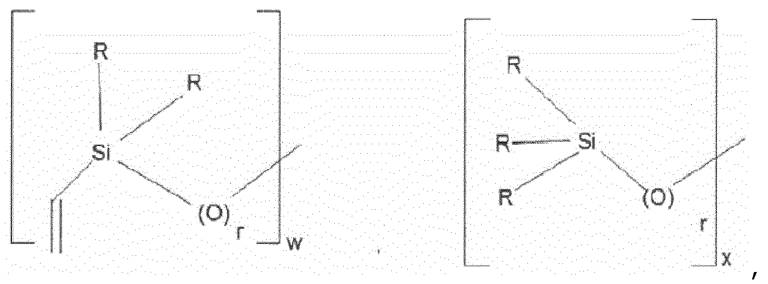 wobei:
a) jedes R unabhängig ein C₁₋₆-Alkyl oder Aryl ist;
b) jedes r unabhängig 0 ist, wenn das Grundgerüst mit Sauerstoff endet, und 1 ist, wenn das Grundgerüst mit Silikon endet;
c) u 0 bis 50 ist, v 5 bis 2.500 ist, w 0 bis 2 ist, x 0 bis 2 ist, w+x=2, u und w nicht gleichzeitig 0 sind, u+v ≥ 1 und u+w mindestens 2 ist.

2. Verfahren zur Herstellung eines Silikonelastomers nach Anspruch 1, wobei 0,05 bis 6 Gewichts-% Hydrid-funktionalisierter Silikonelastomer-Vorläufer verwendet werden.

3. Verfahren zur Herstellung eines Silikonelastomers nach den Ansprüchen 1 oder 2, wobei 5 bis 50 Gewichts-% Vinyl-funktionalisierter Silikonelastomer-Vorläufer verwendet werden.

4. Verfahren zur Herstellung eines Silikonelastomers nach irgendeinem der Ansprüche 1 bis 3, wobei das Gewichtsverhältnis des Hydrid-funktionalisierten Silikonelastomer-Vorläufers zum Vinyl-funktionalisierten Silikonelastomer-Vorläufer größer als 0,015 ist.

5. Verfahren zur Herstellung eines Silikonelastomers nach irgendeinem der Ansprüche 1 bis 4, wobei das Verfahren bei einer Temperatur von 15 bis 75°C durchgeführt wird.

6. Silikonelastomer, erhältlich nach dem Verfahren nach irgendeinem der Ansprüche 1 bis 5.

7. Zusammensetzung für den Endverbrauch, die eine Emulsion darstellt, die 0,001 bis 45 Gewichts-% des Silikonelastomers, erhältlich nach dem Verfahren nach irgendeinem der Ansprüche 1 bis 5, umfasst.

8. Nicht-therapeutische Verwendung der Zusammensetzung nach Anspruch 7 zur Behandlung von Haar, Nägeln und/oder der Haut.

## Revendications

1. Procédé pour la fabrication d'un élastomère de silicone avec actif hydrophobe piégé, comprenant les étapes de :
1. combinaison, dans aucun ordre particulier :
(i) de 0,05 à 8 % en masse d'un précurseur d'élastomère de silicone fonctionnalisé par un hydrure ;
(ii) de 2 à 60 % en masse d'un précurseur d'élastomère de silicone fonctionnalisé par un vinyle ;
(iii) de 0,5 à 97 % en masse d'un solvant comprenant du polydiméthylsiloxane ou de la caprylyltriméthicone ;
(iv) de 0,25 à 65 % en masse d'actif hydrophobe ; et
(v) un catalyseur dans une quantité efficace pour catalyser une polymérisation des précurseurs d'élastomères fonctionnalisés par un hydrure et vinyle ; et de
2. récupération d'élastomère de silicone avec actif hydrophobe piégé, où :
(i) l'actif hydrophobe est soluble dans le solvant ; et
(ii) au moins 20 % de solvant total utilisé pour fabriquer l'élastomère de silicone sont fournis avant que la polymérisation soit initiée où tout reste de solvant utilisé est fourni progressivement en de 5 à 30 minutes dans un gonflement supplémentaire d'élastomère lorsque la polymérisation de l'hydrure est complète à de 90 à 99 % et l'élastomère de silicone avec actif hydrophobe piégé présente une viscosité de 50 à 3 000 cps et un module de stockage G' de 700 à 10 000 PA
(iii) l'actif hydrophobe est un écran solaire, une vitamine soluble dans l'huile, un hydroxyacide, ou le résorcinol ; où le précurseur d'élastomère fonctionnalisé par un hydrure consiste en au moins une unité de squelette des formules : 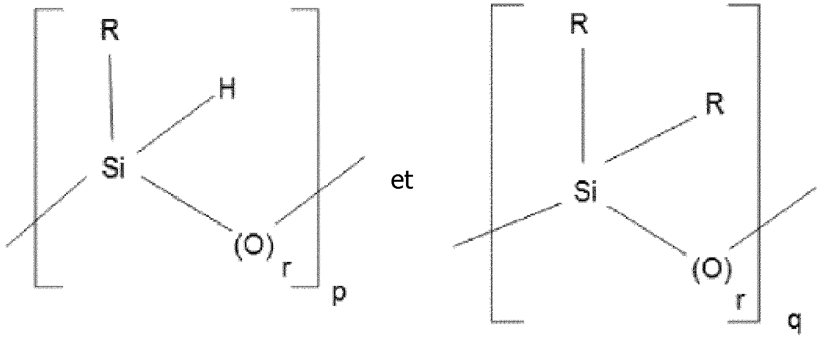 et groupes terminaux des formules : 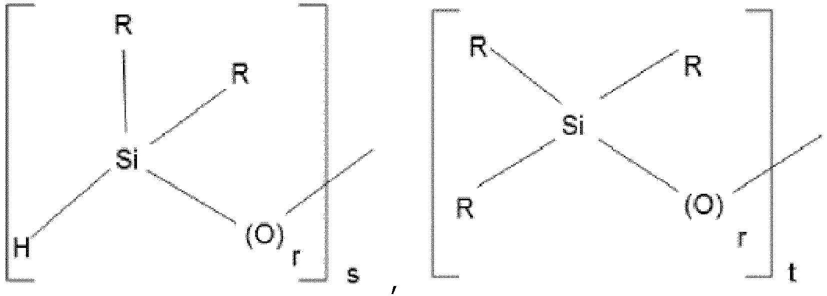 où :
a) chaque R est indépendamment un alkyle en C₁₋₆ ou aryle ;
b) chaque r est indépendamment 0 lorsque l'unité de squelette se termine avec de l'oxygène et 1 lorsque le squelette se termine avec du silicium ;
c) p est de 0 à 50, q est de 2 à 250, s est de 0 à 2, t est de 0 à 2, s+t=2, p et s ne sont pas simultanément 0, p+q≥1 et p+s est d'au moins 2 ;
et où le précurseur d'élastomère fonctionnalisé par un vinyle consiste comme séquences ou arbitrairement dispersée dans celui-ci en au moins une unité de squelette des formules : 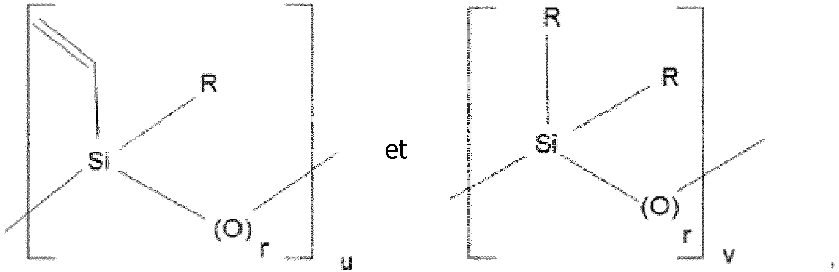 et groupes terminaux des formules : 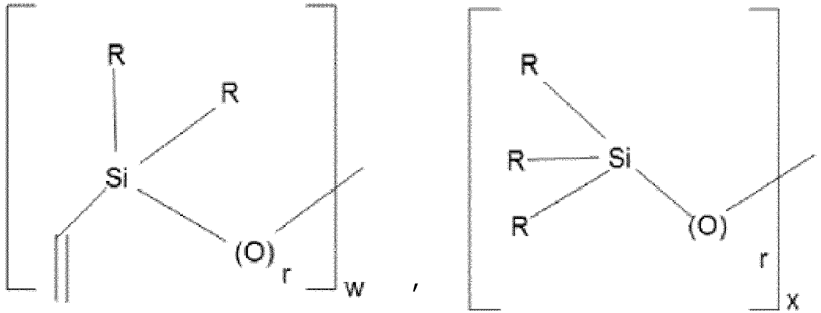 où :
a) chaque R est indépendamment un alkyle en C₁₋₆ ou aryle ;
b) chaque r est indépendamment 0 lorsque le squelette se termine avec de l'oxygène et 1 lorsque le squelette se termine avec du silicium ;
c) u est de 0 à 50, v est de 5 à 2 500, w est de 0 à 2, x est de 0 à 2, w+x=2, u et w ne sont pas simultanément 0, u + v ≥ 1 et u+w est d'au moins 2.

2. Procédé pour la fabrication d'un élastomère de silicone selon la revendication 1, où de 0,05 à 6 % en masse de précurseur d'élastomère de silicone fonctionnalisé par un hydrure sont utilisés.

3. Procédé pour la fabrication d'un élastomère de silicone selon la revendication 1 ou 2, où de 5 à 50 % en masse de précurseur d'élastomère de silicone fonctionnalisé par un vinyle sont utilisés.

4. Procédé pour la fabrication d'un élastomère de silicone selon l'une quelconque des revendications 1 à 3, où le rapport en masse de précurseur d'élastomère de silicone fonctionnalisé par un hydrure au précurseur d'élastomère de silicone fonctionnalisé par un vinyle est supérieur à 0,015.

5. Procédé pour la fabrication d'un élastomère de silicone selon l'une quelconque des revendications 1 à 4, où le procédé est réalisé à une température de 15 à 75°C.

6. Elastomère de silicone pouvant être obtenu par le procédé selon l'une quelconque des revendications 1 à 5.

7. Composition d'utilisation finale qui est une émulsion comprenant de 0,001 à 45 % en masse de l'élastomère de silicone pouvant être obtenu par le procédé selon l'une quelconque des revendications 1 à 5.

8. Utilisation non-thérapeutique de la composition dans la revendication 7 pour traiter les cheveux, les ongles et/ou la peau.
